# EUROPÄISCHE PATENTSCHRIFT

(11) **EP 3 162 492 B1**
(45) Veröffentlichungstag und Bekanntmachung des Hinweises auf die Patenterteilung: **25.07.2018**
(21) Anmeldenummer: 16193261.1
(22) Anmeldetag: 11.10.2016
(51) Int. Cl.: B23K 35/36, C10M 173/02

(54) **VERWENDUNG EINER TRENNMITTELZUSAMMENSETZUNG**
USE OF A RELEASE AGENT COMPOSITION
UTILISATION D'UNE COMPOSITION D'AGENT DE DÉMOULAGE

(30) Priorität: 28.10.2015 AT 6962015
(43) Veröffentlichungstag der Anmeldung: 03.05.2017
(73) Patentinhaber: Hoffmann, Hans, 5026 Salzburg (AT)
(72) Erfinder: Hoffmann, Hans, 5026 Salzburg (AT)
(74) Vertreter: LS-MP von Puttkamer Berngruber Loth Spuhler

(56) Entgegenhaltungen:
- DE-A1-102004 002 608
- DE-C1- 4 426 303
- GB-A- 1 178 049
- KOROLEVA M YU ET AL: "Water mass transfer in W/O emulsions", ANALYTICAL SCIENCES, THE JAPAN SOCIETY FOR ANALYTICAL CHEMISTRY, US, Bd. 297, Nr. 2, 15. Mai 2006 (2006-05-15), Seiten 778-784, XP024909709, ISSN: 0021-9797, DOI: 10.1016/J.JCIS.2005.10.046 [gefunden am 2006-05-15]

## Beschreibung

Die Erfindung bezieht sich auf die Verwendung einer Emulsion, die eine trennwirksame hydrophobe Substanz, wie Öl oder Fett, sowie Wasser und einen Emulgator enthält und als Trennmittel verwendet wird. Eine solche wässrige Emulsion wird als Trennmittel zur Verhinderung des Festsetzens von Schweißspritzern beim Lichtbogenschweißen in DE 44 26 303 C1 beschrieben. Mit der wässrigen Trennmittel-Emulsion können Trennmittel, die aus Lösungen von Ölen oder Fetten in organischen Lösungsmitteln bestehen, ersetzt werden, welche als gesundheitsgefährdend und umweltschädlich angesehen werden.

Bei den wässrigen Trennmittel-Emulsionen besteht jedoch das Problem, dass nach dem Verdunsten des Wassers in dem zurückbleibenden Ölfilm noch eine gewisse Restfeuchtigkeit enthalten ist, die zur Porenbildung in der Schweißnaht führen kann. Gefürchtet ist auch die Einbringung von diffusiblem Wasserstoff durch die Restfeuchte beim Überschweißen der Trennschicht. Die daraus resultierende Gefahr der Rissbildung und von Sprödbrüchen insbesondere beim Schweißen hochfester Stähle ist groß. Der Schweißfachmann ist jedoch angehalten jedes Risiko im Hinblick auf Qualität und Nacharbeit zu vermeiden und ausschließlich abgetrocknete, wasserfreie Trennmittel-Filme zu überschweißen. Die fehlende Möglichkeit der zweifelsfreien Erkennung des wasserfreien Zustandes des Trennmittelfilmes ist ein Grund dafür, dass auch heute noch Trennmittel auf Lösungsmittel-Basis eingesetzt werden.

Außer zum Verhindern von Schweißspritzern beim Schweißen werden Trennmittel auch verwendet, um bei Formverfahren ein Haften der Form mit dem zu formenden Teil zu verhindern. So kann beispielsweise im Betonbau zum Trennen des Betons von der Gießform (Verschalung) eine wässrige Trennmittel-Emulsion verwendet werden, ebenso beispielsweise zum Trennen eines Teils aus Polyurethan-Schaum von der Schaumform.

Während beim Betonbau eine Restfeuchte in dem Trennmittel zur Haftung des Betonteils an der Verschalung führt, reagiert die Isocyanat-Komponente in dem Reaktionsgemisch zur Polyurethanbildung mit Restfeuchtigkeit in der Trennmittelschicht an der Schaumform beispielsweise durch Schaumbildung an der Außenhaut des Schaumstoff-Teils.

Aus GB 1 178 049 A geht eine wässrige Emulsion hervor, die einen Fluoreszenzfarbstoff enthält und zum Aufdecken von Fehlern unterhalb der Oberfläche eines Werkstoffs verwendet wird, beispielsweise von Rissen. DE 4426303 C1 beschreibt die Verwendung einer Emulsion bestehend aus einem Emulgator, einem Öl und Wasser zum Verhindern des Anhaftens von Metallspritzern beim Schweißen. Aufgabe der Erfindung ist es, auf einfache Weise sicherzustellen, dass die Trennmittelschicht keine Restfeuchte enthält.

Dies wird erfindungsgemäß durch einen Farbindikator in der Trennmittelzusammensetzung erreicht, welcher durch einen Farbwechsel der Trennmittelschicht den Übergang von der wasserhaltigen in die wasserfreie Trennmittelschicht anzeigt.

Damit ist die Abtrocknung des wässrigen Trennmittel-Films sicher und problemlos anhand des Farbwechsels erkennbar.

Die erfindungsgemäß verwendete Trennmittelzusammensetzung besteht aus wenigstens einer hydrophoben und damit trennwirksamen Substanz, insbesondere einem Öl vorzugsweise pflanzlicher Natur, beispielsweise aus dem Glycerinester einer gesättigten oder ungesättigten Fettsäure, beispielsweise Rapsöl, ferner einem vorzugsweise nichtionischen Emulgator, wie Dowanol®, und enthält optimal Additive und Inhibitore, z.B. einen Oxidationsinhibitor, ein Korrosionsschutzmittel und/oder ein Biozid, z.B. Fungizid zur Verhinderung der Schimmelbildung. Unter einem Emulgator ist dabei ein häufig auch als Tensid bezeichneter Hilfsstoff zu verstehen.

Der Wassergehalt der Trennmittelzusammensetzung beträgt vorzugsweise 70 bis 99, insbesondere 80 bis 96 Gew.-%.

Der Farbstoffindikator wird durch wenigstens einen wasserlöslichen Tagesleuchtfarbstoff gebildet. Der Tagesleuchtfarbstoff ist vorzugsweise ein Fluoreszenzfarbstoff, der im Wellenlängenbereich des Tageslichts, also zwischen 380 und 780 nm fluoresziert.

Der Tagesleuchtfarbstoff fluoresziert in einer ersten Farbe, wenn die Trennmittelschicht Restfeuchte, also noch Wasser enthält, und weist eine zweite Farbe auf, wenn sie wasserfrei ist.

Die zweite Farbe ist dabei vorzugsweise eine im sichtbaren Licht nicht sichtbare Farbe. Damit der Tagesleuchtfarbstoff im Tageslicht bei feuchtem Trennmittel gut sichtbar ist, jedoch im wasserfreien Zustand der Trennmittelschicht nicht sichtbar ist, also eine farblose Trennmittelschicht gebildet wird, wird vorzugsweise die Menge des Tagesleuchtfarbstoffs in der aufgetragenen Trennmittelschicht begrenzt, beispielsweise auf höchstens 0,05 g, insbesondere 0,003 g Tageleuchtfarbstoff pro m². Das heißt, die wässrige Trennmittelzusammensetzung wird in sehr dünner Schicht, also als Film auf die zu schützende Oberfläche aufgetragen.

Durch die geringe Menge des Farbstoffs können unerwünschte Einflüsse des Trennmittels beispielsweise auf das Schweißen vermieden werden.

Das Auftragen der wässrigen Trennmittelzusammensetzung kann manuell oder automatisch, beispielsweise durch Einsprühen oder Bestreichen der Oberfläche erfolgen, und zwar in einer Menge von vorzugsweise 3 bis 40, insbesondere 10 bis 25 g/m².

Der Feuchtigkeits-Indikator ist zum Zeitpunkt der Applikation in der Wasserphase als Signalfarbe mit freiem Auge bei Tageslicht und bei Arbeitsplatz-Beleuchtung gut erkennbar. Mit dem Grad der Abdunstung der Wasserphase nimmt die Leuchtkraft jedoch ab. Wenn die Wasserphase und damit die Restfeuchte aus dem Trennmittel-Film vollständig entwichen ist, ist auch die Leuchtkraft des Indikators auf der gesamten Applikation vollständig erloschen. Der Anwender hat durch den Farbwechsel somit ein sicheres Signal für den wasserfreien Zustand des Trennmittelfilmes. Die im abgetrockneten Trennmittelfilm verbleibenden Farbpigmente sind bei Tageslicht und bei Arbeitsplatz-Beleuchtung somit mit freiem Auge nicht erkennbar.

Es ist jedoch auch möglich, einen Tageleuchtfarbstoff zu verwenden, der auch bei einem wasserfreien Trennmittelfilm sichtbar ist, jedoch in einer anderen Farbe als bei wasserhaltiger Trennmittelschicht.

Zum Auftragen der Trennmittelschicht wird vorzugsweise eine Trennmittelzusammensetzung verwendet, die 0,01 bis 2, insbesondere 0,05 bis 0,6 g, z.B. 0,1 bis 0,3 g Tagesleuchtfarbstoff je Liter enthält.

Als Tagesleuchtfarbstoff wird vorzugsweise wenigstens ein Farbstoff aus einer Gruppe ausgewählt, die aus Uranin, wenigstens einem Rhodamin, Pyranin, Eosin, Naphtionat, Duasyn® und Tinopal® besteht, wobei als Rhodamin vorzugsweise Rhodamin B, Rhodamin WT, Amidorhodamin G und/oder Sulforhodamin verwendet wird.

Diese Farbstoffe sind als Tagesleuchtstoffe bekannt und werden beispielsweise in der Hydrologie als Fluroeszenz-Tracer eingesetzt.

Dabei haben sich insbesondere folgende handelsübliche Tagesleuchtstoffe als geeignet erwiesen.

| | | | | | |
|---|---|---|---|---|---|
| 1) | Pyranin | 120% | Emission 512 nm | (Lanxess | Deutschland) |
| 2) | Uranin | 75% | Emission 513 nm | (Symrise | Deutschland) |
| 3) | Uranin | 100% | Emission 515 nm | (Fiorio Colori | Italien) |
| 4) | Rhodamin B | 100% | Emission 586 nm | (Fiorio Colori | Italien) |

In der Spalte "Emission" ist die Wellenlänge des Lichts angegeben, bei welcher der Farbstoff das maximale Fluoreszenzsignal emittiert. In Klammern sind die Hersteller der Farbstoffe genannt. Die %-Angaben sind Teil der Handelsbezeichnung.

Zur schweißtechnischen Untersuchung wurden Trennmittelzusammensetzungen mit jeweils 0,2 g/Liter einer Tageleuchtfarbe 1) bis 4) als Farbindikator hergestellt. Als Null-Probe wurde dieselbe Trennmittelzusammensetzung jedoch ohne Farbindikator verwendet.

Die Trennmittelzusammensetzungen mit den Leuchtfarbstoffen 1) bis 4) sind nach der Applikation mittels Handsprühpumpe mit freiem Auge gut erkennbar.

Die erste Überschweißung der Null-Probe und der Proben 1) bis 4) erfolgte unmittelbar nach dem Einsprühen, also im nassen, wasserhaltigen Zustand des Trennmittelfilmes. Für die zweite Versuchsreihe wurde nach dem Einsprühen der Proben die Abtrocknung der Wasserphase abgewartet.

Bei den Proben der mit Tagesleuchtfarbstoffen 1) bis 4) ausgerüsteten Trennmittelzusammensetzung konnte durch Farbumschlag der wasserfreie Zustand des Trennmittelfilmes mit freiem Auge zweifelsfrei erkannt werden.

Überraschenderweise zeigte sich, dass die einzelnen als Indikatoren eingesetzten Tagesleuchtfarbstoffe beim Überschweißen des Trennmittelfilmes unterschiedlich und teilweise deutlich Einfluss nehmen auf das Verhalten des Lichtbogens und auf die Schweißeigenschaft. Im einzelnen wurden Lichtbogeneigenschaft wie folgt erkannt:

| | |
|---|---|
| Probe 0 | Trennmittel ohne Indikator: |
| Nass: | Schweißverhalten gut, Nahtbild gut |
| Trocken: | Schweißverhalten gut, Nahtbild gut |
| | |
| Probe 1 | Indikator Pyranin 120%: |
| Nass: | Schweißverhalten befriedigend (weicherer Lichtbogen), Nahtbild gut |
| Trocken: | Schweißverhalten gut, Nahtbild gut |
| | |
| Probe 2 | Indikator Uranin 75%: |
| Nass: | Schweißverhalten sehr gut, Nahtbild sehr gut |
| Trocken: | Schweißverhalten sehr gut, Nahtbild sehr gut |
| | |
| Probe 3 | Indikator Uranin 100%: |
| Nass: | Schweißverhalten sehr gut, Nahtbild sehr gut |
| Trocken: | Schweißverhalten befriedigend, Nahtbild befriedigend |
| | |
| Probe 4 | Indikator Rhodamin B 100%: |
| Nass: | Schweißverhalten exzellent, Nahtbild exzellent |
| Trocken: | Schweißverhalten exzellent, Nahtbild exzellent |

Danach wird mit der Probe 4, also mit "Rhodamin B 100%", sowohl bei nassem wie bei trockenem Trennmittelfilm das beste Nahtbild und Schweißverhalten erzielt, also eine hervorragende Fokussierung des Lichtbogens.

Dabei dürften das Schweißverhalten und das Nahtbild bei diesen Proben auch durch zusätzliche Stoffe in diesen handelsüblichen Tagesleuchtfarben beeinflusst sein.

Durch Auswahl und Dosierung des Feuchtigkeits-Indikators als Additiv in einem wässrigen Trennmittel gegen Anhaften von Schweißspritzern ist es somit möglich, neben der zweifelsfreien Erkennung des wasserfreien Zustandes des Trennmittelfilms gleichzeitig optional die Eigenschaften des Lichtbogens und die Qualität der Schweißnaht zu beeinflussen.

Die Überschweißungen der Proben 2) und 4) zeigen im Vergleich zur Null-Probe einen äußerst richtungsstabilen, schmalen, druckvollen und spritzerarmen Lichtbogen. Die Schweißnaht zeigt weniger Schuppenbildung, keine Einbrandkerben und eine hervorragende Flankenbenetzung. Durch Auswahl, Dosierung und Mischung der Tagesleuchtfarbstoffe in dem Schweißtrennmittel ist es folglich möglich, gleichzeitig die Lichtbogeneigenschaften beim Überschweißen der Trennmittel-Filme zu modellieren und die Schweißnaht zu optimieren.

Vorzugsweise wird der Indikator oder die Mischung von Indikatoren so ausgewählt, dass kein Einfluss entsteht auf die Eigenschaften des Lichtbogens. Insbesondere werden der Indikator oder die Mischung von Indikatoren so ausgewählt, dass deren Einfluss auf den Lichtbogen zu einer Verbesserung der Schweißergebnisse beim Lichtbogenschweißen führt.

Die erfindungsgemäß verwendete Trennmittelzusammensetzung ist geeignet das Anhaften von Schweißspritzern auf Metallen beim Schweißen, insbesondere beim Lichtbogenschweißen, einschließlich Punkt- und Laserschweißen zu verhindern. Gleiches gilt für das Anhaften von Spritzern aus geschmolzenem Metall beim Laser-Schneiden.

Außer als Trennmittel gegen das Anhaften von geschmolzenen Metallspritzern beim Schweißen oder Laser-Schneiden ist die erfindungsgemäß verwendete Trennmittelzusammensetzung auch beispielsweise zum Trennen von einer Gießform geeignet. Das heißt, erfindungsgemäß kann z.B. verhindert werden, dass der Beton durch Feuchtigkeit in der Trennmittelbeschichtung der Gießform (Verschalung) nach dem Aushärten an der Gießform bzw. Verschalung haftet.

Für Sichtbeton werden z.B. überwiegend Schalungen mit Kunststoffschalhaut eingesetzt, die meist hellgrau ist. Die Erkennung des wasserfreien Zustandes des Trennmittelfilmes ist damit kaum zweifelsfrei möglich.

Zudem kann die erfindungsgemäß verwendete Trennmittelzusammensetzung zum Trennen eines Teils aus Polyurethan, insbesondere einem Polyurethan-Schaumstoff nach dem Formen von der Form verwendet werden.

Das heißt, wenn des Tagesleuchtfarbstoff eine trockene Trennmittelschicht anzeigt, kann verhindert werden, dass die Isocyanat-Komponente in dem Reaktionsgemisch zur Polyurethan-Bildung durch Reaktion mit Wasser, also mit Restfeuchtigkeit in der Trennmittelschicht an der Form bzw. Schaumform reagiert, so dass das gebildete Teil aus massiven Polyurethan bzw. Polyurethan-Schaumstoff mit der Form bzw. Schaumform verklebt, wobei sich darüber hinaus durch Reaktion der Isocyanat-Komponente des Reaktionsgeschmischs zur Polyurethan-Bildung mit der Restfeuchte in der Trennmittelschicht Blasen aus Kohlendioxid in dem massiven Polyurehtan-Teil bzw. in der Außenhaut bilden können, ebenso in der Außenhaut des aus Polyurethan-Schaumstoff gebildeten Teils.

Wegen der vorzugsweise geringen Dosierungsmenge der Indikatoren von vorzugsweise 0,01 bis 2 g, insbesondere 0,1 bis 0,3 g/Liter Fertigprodukt ist es vorteilhaft, die Dosierung mit einer Farbindikatorlösung in Form eines beispielsweise 10-prozentigen Konzentrates vorzunehmen. Die Lösung des wenigstens einen Farbindikators zu dem Konzentrat erfolgt vorzugsweise mit Wasser und optional mit Zugabe von polaren Lösungsmitteln, beispielsweise ein- oder mehrwertigen Alkoholen. Eine vorteilhafte Variante ist es, das Farbindikator-Konzentrat mit einer Teilmenge der ausgewählten wässrigen Trennmittelzusammensetzung herzustellen. Das Indikator-Konzentrat wird in der gewünschten Dosierung der wässrigen Trennmittelzusammensetzung zugegeben und durch Rühren, Schütteln oder dergleichen homogen eingearbeitet. Das Fertigprodukt wird vorzugsweise in Behältern vor Licht geschützt, um das Ausbleichen des Farbindikators oder der Farbindikatoren-Mischung zu verhindern.

## Patentansprüche

1. Verwendung einer Zusammensetzung als Trennmittel, die eine trennwirksame Substanz, Wasser, einen Emulgator und einen Farbindikator enthält, welcher durch Farbwechsel den Übergang von einer wasserhaltigen in eine wasserfreie Trennmittelschicht anzeigt.

2. Verwendung nach Anspruch 1, **dadurch gekennzeichnet, dass** der Farbindikator wenigstens ein wasserlöslicher Tagesleuchtfarbstoff ist.

3. Verwendung nach Anspruch 1 oder 2, **dadurch gekennzeichnet, dass** der Wassergehalt der Zusammensetzung 70 bis 99 Gew.-% beträgt.

4. Verwendung nach Anspruch 2 oder 3, **dadurch gekennzeichnet, dass** die Zusammensetzung 0,01 bis 2 g Tagesleuchtfarbstoff je Liter enthält.

5. Verwendung nach einem der Ansprüche 2 bis 4, **dadurch gekennzeichnet, dass** die mit der Zusammensetzung gebildete Trennmittelschicht höchstens 0,05 g des wenigstens einen Tagesleuchtfarbstoffs pro m² aufweist.

6. Verwendung nach einem der Ansprüche 2 bis 5, **dadurch gekennzeichnet, dass** der wenigstens eine Tagesleuchtfarbstoff aus einer Gruppe ausgewählt ist, die aus Uranin, Pyranin und Eosin besteht, aus der Rhodamin-Gruppe ausgewählt ist oder ein Naphtionat ist.

7. Verwendung nach Anspruch 6, **dadurch gekennzeichnet, dass** das Rhodamin Rhodamin B, Rhodamin WT, Amidorhodamin G und/oder Sulforhodamin ist.

8. Verwendung nach einem der vorstehenden Ansprüche zum Verhindern des Anhaftens von geschmolzenen Metallspritzern beim Schweißen oder Laser-Schneiden.

9. Verwendung nach einem der Ansprüche 1 bis 7 zum Trennen von Beton von einer Gießform.

10. Verwendung nach einem der Ansprüche 1 bis 7 zum Trennen eines Teils aus Polyurethan oder Polyurethan-Schaum nach dem Formen von der Form.

## Claims

1. Use as a release agent of a composition containing a substance having a release effect, water, an emulsifying agent and a color indicator indicating the transition from a hydrous to an anhydrous release agent layer by color change.

2. Use according to claim 1, **characterized in that** the color indicator is at least one watersoluble daylight fluorescent dye.

3. Use according to claim 1 or 2, **characterized in that** the water content of the composition is 70 to 99 % by weight.

4. Use according to claim 2 or 3, **characterized in that** the composition contains 0.01 to 2 g of daylight fluorescent dye per liter.

5. Use according to any of claims 2 to 4, **characterized in that** the release agent layer formed with the composition comprises not more than 0.05 g of the at least one daylight fluorescent dye per m².

6. Use according to any of claims 2 to 5, **characterized in that** the at least one daylight fluorescent dye is selected from a group consisting of uranine, pyranine and eosine, is selected from the rhodamine group or is a naphtionate.

7. Use according to claim 6, **characterized in that** the rhodamine is rhodamine B, rhodamine WT, amidorhodamine G and/or sulforhodamine.

8. Use according to any of the preceding claims for preventing adhesion of molten metal splashes during welding or laser cutting.

9. Use according to any of claims 1 to 7 for releasing concrete from a casting mold.

10. Use according to any of claims 1 to 7 for releasing a part made of polyurethane or polyurethane foam from the mold after molding.

## Revendications

1. Utilisation d'une composition en tant qu'agent de démoulage, qui contient une substance de démoulage active, de l'eau, un agent émulsionnant et un indicateur de couleur qui indique par le changement de couleur le passage d'une couche d'agent de démoulage contenant de l'eau en une couche d'agent de démoulage anhydre.

2. Utilisation selon la revendication 1, **caractérisée en ce que** l'indicateur de couleur est au moins un colorant fluorescent à la lumière du jour soluble dans l'eau.

3. Utilisation selon les revendications 1 ou 2, **caractérisée en ce que** la teneur en eau de la composition est de 70 à 99 % en poids.

4. Utilisation selon les revendications 2 ou 3, **caractérisée en ce que** la composition contient de 0,01 à 2 g de colorant fluorescent à la lumière du jour par litre.

5. Utilisation selon l'une des revendications 2 à 4, **caractérisée en ce que** la couche d'agent de démoulage formée avec la composition présente tout au plus 0,05 g dudit au moins un colorant fluorescent à la lumière du jour par m².

6. Utilisation selon l'une des revendications 2 à 5, **caractérisée en ce que** ledit au moins un colorant fluorescent à la lumière du jour est choisi parmi un groupe qui est constitué par la fluorescéine, la pyranine et l'éosine, est choisi parmi un groupe de rhodamine ou est un naphtionate.

7. Utilisation selon la revendication 6, **caractérisée en ce que** la rhodamine est de la rhodamine B, de la rhodamine WT, de l'amidorhodamine G et/ou de la sulforhodamine.

8. Utilisation selon l'une des revendications précédentes pour empêcher l'adhérence de projections de métal fondu lors du soudage ou du découpage au laser.

9. Utilisation selon l'une des revendications 1 à 7 pour séparer du béton d'un moule.

10. Utilisation selon l'une des revendications 1 à 7 pour séparer d'un moule une pièce de polyuréthane ou de mousse en polyuréthane après le moulage.
